# EUROPÄISCHE PATENTSCHRIFT

(11) **EP 4 395 878 B1**
(45) Veröffentlichungstag und Bekanntmachung des Hinweises auf die Patenterteilung: **26.02.2025**
(21) Anmeldenummer: 23717571.6
(22) Anmeldetag: 12.04.2023
(51) Int. Cl.: A61N 1/05, A61N 1/39

(54) **ELEKTROMEDIZINISCHE ELEKTRODENANORDNUNG**
ELECTROMEDICAL ELECTRODE ASSEMBLY
ENSEMBLE ÉLECTRODE ÉLECTROMÉDICALE

(30) Priorität: 21.04.2022 DE 102022109680
(43) Veröffentlichungstag der Anmeldung: 10.07.2024
(73) Patentinhaber: Osypka AG, 79618 Rheinfelden-Baden (DE)
(72) Erfinder: OSYPKA, Peter, 79639 Grenzach-Wyhlen (DE); OSYPKA, Nicola, 82237 Wörthsee (DE); KITSCHMANN, Achim, 4057 Basel (CH); SCIUTO, Francesco, 79650 Schopfheim (DE)
(74) Vertreter: Mertzlufft-Paufler, Cornelius
(86) Internationale Anmeldenummer: PCT/EP2023/059541
(87) Internationale Veröffentlichungsnummer: WO 2023/202925

(56) Entgegenhaltungen:
- EP-A2- 0 280 564
- DE-A1- 102011 111 649
- DE-A1- 102017 008 720

## Beschreibung

Die Erfindung betrifft eine elektromedizinische Elektrodenanordnung mit zumindest einer implantierbaren Kardioversionselektrode zur Abgabe von elektrischen Impulsen und Kardioversionsimpulsen auf ein Zielgewebe, insbesondere auf Herzmuskelgewebe.

Die DE 10 2017 008 720 A1 offenbart eine bipolare Elektrodenvorrichtung, bestehend aus einem Stimulationspol mit einer isolierten Litze, die proximal zum externen Schrittmacher geführt wird und somit die elektrische Zuleitung darstellt; distal zum Stimulationspol befindet sich ein Fixierungselement sowie die zur Implantation benötigte Herznadel oder ein Loop. Die Elektrodenvorrichtung besteht zudem aus einem Kardioversionspol mit einer isolierten Litze, wobei der Kardioversionspol aus blanker wellenförmig verlaufender Litze besteht, ein distales und ein proximales Ende aufweist, proximal die isolierte Litze zum externen Schrittmacher geführt wird und somit die elektrische Zuleitung darstellt. Distal wird die Litze isoliert weitergeführt wird, wobei die isolierten Litzen parallel geführt werden und die isolierte Litze am Kardioversionspol vorbeigeführt wird. Die Elektrodenanordnung ist dadurch gekennzeichnet, dass der Kardioversionspol proximal und distal durch thermisches Verbinden der isolierten Litzen und fixiert ist.

Die EP 0 280 564 A2 offenbart ein Paar von Defibrillations-Patchelektroden zur Platzierung am Herzen, um Defibrillationsschocks abzugeben, umfassend: eine erste Patchelektrode, die so geformt ist, dass sie über die rechte Herzkammer passt, eine zweite Patchelektrode, die so geformt ist, dass sie über die linke Herzkammer passt, und zwar in einem Abstand zur ersten Patchelektrode, so dass ein Spalt zwischen den einander gegenüberliegenden Rändern der beiden Patchelektroden gebildet wird, wobei jede der Patchelektroden eine Einrichtung zur Abgabe der Defibrillationsschocks an die jeweiligen Elektroden aufweist, und wobei jede der Patchelektroden eine Größe und Form aufweist, um einen wesentlichen Teil des jeweiligen ventrikulären Myokards innerhalb der Grenzen der jeweiligen Patchelektrode zu umfassen, um ein im Wesentlichen gleichmäßiges Potentialgradientenfeld über die gesamte ventrikuläre Masse aufzubauen, wenn ein Defibrillationsschock an die Elektroden abgegeben wird.

Die DE 10 2011 111 649 A1 offenbart eine Ausführung und Fixierung einer implantierbaren epikardialen Elektrodenanordnung, die eine Messung, Überwachung, Stimulation und Defibrillation des Herzens beim Menschen in Verbindung mit einem außerhalb des Körpers anschließbaren Herzschrittmacher oder Defibrillator ermöglicht und nach erfolgter Anwendung jederzeit leicht durch Zug entfernbar ist, wobei die Elektrodenanordnung die Form eines Tennisschlägers besitzt.

Derartige elektromedizinische Elektrodenanordnungen werden Patienten beispielsweise nach einer Herzoperation temporär implantiert und zur Unterstützung der Herztätigkeit verwendet. Über die implantierbare Kardioversionselektrode ist es möglich, im Bedarfsfall Kardioversionsimpulse auf das Herz des Patienten abzugeben, um Reizleitungsstörungen zu beheben. Im Vergleich zu Pacing- oder Schrittmacherimpulsen, die abgegeben werden, um einen bestimmten Herzrhythmus vorzugeben oder zu unterstützen, sind derartige Kardioversionsimpulse stärker. Um Verletzungen am Zielgewebe zu vermeiden, dürfen über Elektroden, die ausschließlich für die Abgabe von Schrittmacherimpulsen vorgesehen sind, keine Kardioversionsimpulse abgegeben werden.

Aufgrund ihrer Stärke werden Kardioversionsimpulse von Patienten zudem oftmals als schmerzhaft empfunden. Dies insbesondere dann, wenn die Kardioversionsimpulse über externe Elektroden abgegeben werden. Aber auch über implantierbare Kardioversionselektroden abgegebene Kardioversionsimpulse können für Patienten als unangenehm, wenn nicht gar als schmerzhaft empfunden werden. Eine weitere Schwierigkeit bei der Abgabe von Kardioversionsimpulsen über implantierbare Kardioversionselektroden besteht darin, das Zielgewebe für eine erfolgreiche Kardioversion ausreichend zu stimulieren. Auf die Wirksamkeit der Kardioversionsimpulse kann der Kontakt der Kardioversionselektrode zum Zielgewebe einen wichtigen Einfluss haben.

Aufgabe der Erfindung ist es, eine Elektrodenanordnung der eingangs genannten Art mit verbesserten Gebrauchseigenschaften bereitzustellen.

Zur Lösung der Aufgabe wird eine elektromedizinische Elektrodenanordnung mit zumindest einer implantierbaren Kardioversionselektrode zur Abgabe von Kardioversionsimpulsen auf ein Zielgewebe, insbesondere auf Herzmuskelgewebe, vorgeschlagen, die die Mittel und Merkmale des unabhängigen, auf eine derartige Elektrodenanordnung gerichteten Anspruchs aufweist. Zur Lösung der Aufgabe wird somit bei der eingangs definierten Elektrodenanordnung insbesondere vorgeschlagen, dass die Kardioversionselektrode der elektromedizinischen Elektrodenanordnung aus einem flexiblen, in unverformter Gebrauchsstellung ebenen, elektrisch leitenden Flächengebilde besteht, das zur flächigen Anlage an ein Zielgewebe eingerichtet ist.

Ein derartiges Flächengebilde lässt sich besonders zuverlässig an das Zielgewebe des zu behandelnden Herzens anlegen. Das Flächengebilde kann im Körper des zu behandelnden Patienten beispielsweise an eine Außenseite des Herzens, beispielsweise an die Außenseite eines Vorhofs des zu behandelnden Herzens, angelegt werden. Das Flächengebilde kann hierbei einen vergleichsweise großflächigen Kontakt zu dem zu behandelnden Zielgewebe herstellen und sich zuverlässig an die in der Regel gekrümmten Oberflächen des zu behandelnden Zielgewebes anlegen lassen. Auf diese Weise kann ein für eine schonende und sichere Kardioversion ausreichender Kontakt zwischen der Kardioversionselektrode der Elektrodenanordnung und des zu behandelnden Gewebes hergestellt werden. Dies begünstigt einerseits die Effektivität der Kardioversionsbehandlung und andererseits erlaubt die großflächige Anlage die Abgabe von Kardioversionsimpulsen auf eine vergleichsweise große Fläche. Dadurch kann das Flächengebilde die Kardioversion mit Kardioversionsimpulsen geringerer Intensität begünstigen. Kardioversionsimpulse geringerer Intensität werden in der Regel als weniger schmerzhaft empfunden und verursachen oftmals weniger Nebenwirkungen. So können sich im besten Fall Schmerzen bei der Kardioversionsbehandlung verringern oder gar vollständig vermeiden lassen. Insgesamt begünstig die erfindungsgemäße Elektrodenanordnung somit die effektive und gleichzeitig schonende Kardioversion mittels einer implantierten Kardioversionselektrode. Ferner zeichnet sich die erfindungsgemäße Elektrodenanordnung durch ihre vereinfachte Handhabung und Anwendung am Patienten aus. Die Elektrodenanordnung kann einfach appliziert werden und erlaubt die Verwendung eines elektromedizinischen Impulsgebers, nämlich eines Pacers und/oder Kardioverters am Patientenbett.

Aufgrund des Umstandes, dass die Kardioversionselektrode der Elektrodenanordnung aus einem flexiblen Flächengebilde besteht, ist es außerdem möglich, die Kardioversionselektrode, beispielsweise durch einen Zug an einem proximalen Ende der Kardioversionsanordnung, schonend zu explantieren. Bei der Explantation kann sich das Flächengebilde, beispielsweise durch einen Zug am proximalen Ende der Elektrodenanordnung, derart strecken und dabei verformen, dass bei der Explantation eine quer zu einer Explantationsrichtung ausgerichtete Ausdehnung des Flächengebildes reduziert wird. Dies begünstigt eine atraumatische Explantation der Kardioversionselektrode.

Bei einer Ausführungsform der Elektrodenanordnung weist das Flächengebilde einen elektrischen Widerstand von weniger als 150 Ohm, vorzugsweise von weniger als 70 Ohm, auf. Dies kann die Abgabe ausreichend starker Kardioversionsimpulse begünstigen und das zu behandelnde Zielgewebe vor Verletzungen schützen.

Das Flächengebilde kann eine Längsachse, insbesondere eine Längsmittelachse aufweisen, die bei unverformtem Flächengebilde in einer Ebene verläuft. Das Flächengebilde ist in unverformter Ausgangstellung somit eben, jedoch aufgrund seiner Flexibilität derart verformbar, dass es an ein zu behandelndes Zielgewebe flächig angelegt werden kann.

Das Flächengebilde kann bei einer Ausführungsform der Elektrodenanordnung aus einer longitudinalen Struktur, beispielsweise aus einem elektrischen Leiter, mit wechselnder Biegerichtung bestehen und/oder sinusförmig sein. Die longitudinale Struktur kann ein elektrischer Leiter, beispielsweise eine Litze, sein. Der Leiter kann gebogen sein, beispielsweise sinusförmig gebogen sein, um das Flächengebilde der Kardioversionselektrode zu formen. Insbesondere im Bereich des Flächengebildes der Kardioversionselektrode ist der Leiter vorzugsweise nicht isoliert, so dass eine Abgabe von Kardioversionsimpulsen über den das Flächengebilde bildenden Leiter möglich ist.

Dadurch, dass das Flächengebilde sinusförmig gestaltet sein und/oder aus einer longitudinalen Struktur, beispielsweise aus einem Leiter, der mit wechselnder Biegerichtung gebogen ist, bestehen kann, kann das Flächengebilde nicht nur flexibel, sondern auch elastisch sein. Die Elastizität des Flächengebildes kann die zuverlässige Anlage des Flächengebildes an ein Zielgewebe begünstigen.

Bei einer bevorzugten Ausführungsform der Elektrodenanordnung ist das Flächengebilde aus genau einem elektrischen Leiter gebildet. Es ist aber auch möglich, dass das Flächengebilde aus mehreren elektrischen Leitern zur Abgabe von Kardioversionsimpulsen gebildet ist.

Der genau eine oder der zumindest eine Leiter, aus dem das Flächengebilde gebildet sein kann, kann einen elektrischen Widerstand aufweisen, der kleiner als 150 Ohm, vorzugsweise kleiner als 70 Ohm ist. Auf diese Weise kann ein Flächengebilde mit einem elektrischen Widerstand, der kleiner als 150 Ohm, vorzugsweise kleiner als 70 Ohm ist, aus dem Leiter gebildet werden.

Der Leiter kann eine Längsachse, insbesondere eine Längsmittelachse, aufweisen, die bei unverformtem Flächengebilde in einer Ebene, beispielsweise in einer Mittelebene, des Flächengebildes und/oder mit wechselnder Biegerichtung verläuft. Auf diese Weise kann aus dem elektrischen Leiter ein Flächengebilde mit einer Form bereitgestellt sein, die als Zick-Zack-Form beschrieben werden kann. Bei einer Ausführungsform der Elektrodenanordnung kann der elektrische Leiter mit wechselnder Biegerichtung zu dem Flächengebilde gebogen sein. Die wechselnde Biegerichtung des elektrischen Leiters kann dem Flächengebilde seine Flexibilität und/oder Elastizität verleihen. Der Leiter kann zur Ausbildung eines sinusförmigen Flächengebildes sinusförmig gebogen sein.

Der elektrische Leiter kann einen Durchmesser D von beispielsweise 0,1-0,5 mm aufweisen. Bei einer Ausführungsform kann der Leiter einen Durchmesser D von 0,1-0,3 mm, beispielsweise einen Durchmesser D von 0,2 mm aufweisen.

Der elektrische Leiter kann zumindest zwei aneinander angrenzende und/oder miteinander verbundene Leiterabschnitte aufweisen, die gemeinsam einen Winkel α zwischen 10° und 170° aufspannen. Der Leiter kann zumindest zwei aneinander angrenzende Leiterabschnitte aufweisen, die gemeinsam einen Winkel α zwischen 30° und 90° oder auch einen Winkel α zwischen 30° und 60° oder auch einen Winkel α von 35° aufspannen.

Zwei aneinander angrenzende Leiterabschnitte des Leiters, können durch einen Biegeradius von beispielsweise 0,3 mm bis 2 mm miteinander verbunden sein.

Das Flächengebilde kann in nicht aufgespanntem Zustand eine in Richtung seiner Längsachse messbare Längenausdehnung L1 zwischen jeweils einschließlich 10-50 mm, insbesondere zwischen jeweils einschließlich 25-40 mm, aufweisen. Das Flächengebilde kann in nicht aufgespanntem Zustand eine quer zu seiner Längsachse messbare Ausdehnung L2 von jeweils einschließlich 5-30 mm haben und/oder aus zumindest einem Leiter gebildet sein, dessen Länge 40-140 mm beträgt.

Das Flächengebilde kann als zumindest einen elektrischen Leiter eine abisolierte Litze zur Abgabe von Kardioversionsimpulsen aufweisen.

Das Flächengebilde, insbesondere der elektrische Leiter, kann beispielsweise aus Edelstahl, aus Platin, aus einer Platinlegierung, aus Gold, aus einer Goldlegierung, aus Magnesium, aus einer Magnesiumlegierung, aus Molybdän oder auch aus einer Molybdänlegierung bestehen. Die Verwendung von Edelstahl ist besonders bevorzugt, da dieses Material seit Jahrzehnten am Patienten erprobt, für den Menschen als unbedenklich befunden und zudem aufgrund seiner Belastbarkeit, Flexibilität und Elastizität für den Anwendungsfall zur Herstellung des zuvor erläuterten Flächengebildes besonders geeignet ist.

Erfindungsgemäß weist die Elektrodenanordnung zwei Fixiermittel auf, zwischen denen das Flächengebilde angeordnet ist. Die Fixiermittel der Elektrodenanordnung sind zur Befestigung und/oder Aufspannung des Flächengebildes an einem Zielgewebe, insbesondere an einer Herzaußenseite, eingerichtet. Mithilfe der beiden Fixiermittel der Elektrodenanordnung kann das Flächengebilde als Kardioversionselektrode an einer Außenseite eines zu behandelnden Zielgewebes flächig in Anlage gebracht und dort gehalten werden. Insbesondere dann, wenn das Flächengebilde nicht nur flexibel, sondern auch elastisch ist, kann das Flächengebilde mithilfe der beiden Fixiermittel in seiner Gebrauchsstellung an einem zu behandelnden Zielgewebe aufgespannt werden. Durch die Elastizität kann sich das Flächengebilde dann gegen die Rückhaltekraft der beiden Fixiermittel selbsttätig an das Zielgewebe anlegen und angelegt halten und so einen zuverlässigen Kontakt zu dem zu stimulierenden Zielgewebe sicherstellen. Die beiden Fixiermittel begünstigen somit die zuverlässige Anlage des als Kardioversionselektrode dienenden Flächengebildes der Elektrodenanordnung an ein schlagendes Herz. Die flächige Anlage des Flächengebildes unter Spannung kann das Ablösen des Flächengebildes oder auch nur von Teilen des Flächengebildes verhindern. Dies begünstigt eine zuverlässige und schonende Kardioversionsbehandlung mithilfe der Elektrodenanordnung.

Bei einer Ausführungsform der Elektrodenanordnung weist das Flächengebilde zu zumindest einem der beiden Fixiermittel oder auch zu beiden Fixiermitteln jeweils einen Abstand von zwischen einschließlich 10 und 70 mm auf. Wie zuvor erwähnt, kann das Flächengebilde, insbesondere durch die zuvor erwähnten Fixiermittel, an einem Zielgewebe aufspannbar und/oder an eine Vorhofgröße eines zu behandelnden Herzens anpassbar sein. Aufgespannt kann das Flächengebilde eine Fläche beispielsweise zwischen 30 mm² und 1600 mm² abdecken. Die mit dem Flächengebilde überdeckte Fläche kann sich aus dem Produkt von Längserstreckung und Quererstreckung des aufgespannten Flächengebildes berechnen.

Ein distales Fixiermittel der Elektrodenanordnung zur Befestigung und/oder Aufspannung des Flächengebildes kann distal des Flächengebildes angeordnet sein. Beispielsweise kann das Flächengebilde an einem distalen Ende der Elektrodenanordnung oder zwischen einem distalen Ende der Elektrodenanordnung und dem Flächengebilde angeordnet sein.

Grundsätzlich ist es möglich, das Fixiermittel mit Nahtmaterial im Zielgewebe zu positionieren, um das Fixiermittel im Zielgewebe zu befestigen und die Elektrodenanordnung so zu verankern. Dies kann jedoch vergleichsweise aufwendig sein, da das Nahtmaterial zunächst mit dem Fixiermittel verbunden werden muss.

Um diesen Aufwand zu vermeiden, kann die Elektrodenanordnung an ihrem distalen Ende zumindest ein Positioniermittel, beispielsweise eine Herznadel, zur Positionierung eines Fixiermittels, insbesondere des zuvor erwähnten Fixiermittels, im Zielgewebe aufweisen. Mithilfe des Positioniermittels kann das Fixiermittel zur Verankerung Zielgewebe dort positioniert werden. Nach erfolgreicher Positionierung kann das Positioniermittel abgetrennt und entfernt werden.

Die Elektrodenanordnung kann eine Anschlussleitung aufweisen, über den das Flächengebilde mit einem Stimulationsgeber verbindbar ist. Die Elektrodenanordnung kann zu diesem Zweck ein Anschlussmittel aufweisen, das am proximalen Ende der Anschlussleitung angeordnet oder ausgebildet ist. Die Anschlussleitung kann an einem Punkt mit dem Flächengebilde verbunden sein, der auf einer Längsachse, insbesondere auf einer Längsmittelachse des Flächengebildes liegt.

Die Anschlussleitung kann als Zugmittel verwendet werden, mit dessen Hilfe das als Kardioversionselektrode fungierende Flächengebilde durch einen Zug aus seiner implantierten Gebrauchsstellung explantiert werden kann. Wenn die Anschlussleitung dabei an einem Punkt mit dem Flächengebilde verbunden ist, der auf einer Längsmittelachse des Flächengebildes liegt, kann das Flächengebilde bei der Explantation durch den Zug derart verformt werden, dass sich eine quer zur Zugrichtung ausgerichtete und vorzugsweise gleichmäßige Querschnittsreduktion des Flächengebildes ergibt. Dies kann eine atraumatische Explantation des Flächengebildes begünstigen.

Bei einer Ausführungsform der Elektrodenanordnung ist vorgesehen, dass diese zumindest eine Lateral-Elektrode zur Abgabe von Pacing-Impulsen, die auch als Schrittmacherimpulse bezeichnet werden können, aufweist.

Beispielsweise im Zusammenspiel mit der Kardioversionselektrode können so mithilfe der Lateral-Elektrode auch Pacing-Impulse auf das zu behandelnde Herz abgegeben werden. Somit kann die Elektrodenanordnung dann eine Doppelfunktion übernehmen und nicht nur Kardioversionsimpulse, sondern auch Pacing-Impulse auf das Herz abgeben.

Der zumindest einen Lateral-Elektrode der Elektrodenanordnung kann zumindest ein Fixiermittel zur Befestigung der Lateral-Elektrode an oder in einem Zielgewebe zugeordnet sein. Auf diese Weise ist es möglich, die Lateral-Elektrode für die Zeit, in der die Elektrodenanordnung zur Unterstützung der Herzfunktion eines Patienten verwendet werden soll, zuverlässig im Zielgewebe zu verankern.

Bei einer besonders vorteilhaften Ausführungsform der Elektrodenanordnung ist vorgesehen, dass das Fixiermittel, das der Lateral-Elektrode zugeordnet ist, ein Fixiermittel von zumindest zwei Fixiermitteln zur Befestigung und/oder zur Aufspannung des Flächengebildes ist.

Auf diese Weise hat das der Lateral-Elektrode zugeordnete Fixiermittel also eine Doppelfunktion. Einerseits wird das Fixiermittel zur Verankerung der Lateral-Elektrode im Zielgewebe verwendet, andererseits dient es auch dazu, das als Kardioversionselektrode dienende Flächengebilde zuverlässig in seiner Gebrauchsstellung zu halten. Bei dieser Ausführungsform der Elektrodenanordnung kann somit auf ein separates, nur zur Fixierung der Lateral-Elektrode dienendes Fixiermittel verzichtet werden.

Zur zuverlässigen Fixierung der Lateral-Elektrode und der Kardioversionselektrode, nämlich des Flächengebildes, ist bei dieser Ausführungsform der Elektrodenanordnung nur ein Minimum an Fixiermitteln vorgesehen. Auf diese Weise werden durch die Fixiermittel verursachte Läsionen am Zielgewebe auf ein Minimum beschränkt.

Die zumindest eine Lateral-Elektrode und die zumindest eine Kardioversionselektrode können in einem frei wählbaren Winkel relativ zueinander anordenbar sein. Bei einer bevorzugten Ausführungsform der Elektrodenanordnung spannen die Lateral-Elektrode und die Kardioversionselektrode gemeinsam einen Winkel zwischen 1° und 170° auf. Die zuvor erwähnte Anschlussleitung, die Lateral-Elektrode und die Kardioversionselektrode können bei einer Ausführungsform der Elektrodenanordnung in implantierter Gebrauchsstellung gemeinsam eine Y-Form bilden.

Die Elektrodenanordnung, insbesondere die zumindest eine Kardioversionselektrode und/oder die zumindest eine Lateral-Elektrode können durch Zug an einem proximalen Ende der Elektrodenanordnung, insbesondere an einem proximalen Ende der zuvor bereits erwähnten Anschlussleitung, explantierbar sein.

Der Leiter, aus dem das Flächengebilde bestehen kann, kann ein elektrischer Leiter der Anschlussleitung sein. Das Flächengebilde kann somit aus einem elektrischen Leiter, insbesondere aus einer Litze, der Anschlussleitung bestehen.

Nachfolgend werden Ausführungsbeispiele der Erfindung anhand der Zeichnung näher beschrieben. Die Erfindung ist hierbei nicht auf die gezeigten Ausführungsbeispiele beschränkt. Weitere Ausführungsbeispiel der Erfindung ergeben sich durch Kombination der Merkmale der Ansprüche und/oder durch Kombination der Merkmale der in den Figuren gezeigten Ausführungsbeispiele. Es zeigen:
- Figur 1: eine Seitenansicht einer ersten Ausführungsform einer Elektrodenanordnung mit einer implantierbaren Kardioversionselektrode, die aus einem flexiblen, in unverformter Ausgangstellung ebenen, elektrisch leitenden Flächengebilde besteht, das zur flächigen Anlage an ein Zielgewebe eingerichtet ist,
- Figur 2:: eine Variante der in Figur 1 gezeigten Elektrodenanordnung, mit einer zusätzlich zu der Kardioversionselektrode vorhandenen Lateral- Elektrode, über die Pacing-Impulse auf ein Zielgewebe abgegeben werden können, sowie
- Figur 3:: eine vergrößerte Detailansicht des Flächengebildes der in den Figuren 1 und 2 gezeigten Elektrodenanordnungen.

Sämtliche Figuren zeigen zumindest Teile von im Ganzen mit 1 bezeichneten elektromedizinische Elektrodenanordnungen. Jede gezeigte Elektrodenanordnung 1 ist zur Abgabe elektrischer Impulse auf ein Zielgewebe, nämlich auf Herzmuskelgewebe, eingerichtet. Jede gezeigte Elektrodenanordnung 1 weist eine implantierbare Kardioversionselektrode 2 zur Abgabe von Kardioversionsimpulsen auf.

Die Kardioversionselektroden 2 bestehen jeweils aus einem flexiblen, in unverformter Ausgangstellung ebenen, elektrisch leitenden Flächengebilde 3, das zur flächigen Anlage an ein Zielgewebe eingerichtet ist. Das Flächengebilde 3 ist elastisch und weist einen elektrischen Widerstand von weniger als 150 Ohm, vorzugsweise von weniger als 70 Ohm auf.

Das Flächengebilde 3 weist eine Längsachse 4, nämlich eine Längsmittelachse auf, die bei unverformtem Flächengebilde 3 in einer Ebene verläuft. Das Flächengebilde 3 ist aus einer longitudinalen Struktur, nämlich aus einem elektrischen Leiter 5 mit wechselnder Biegerichtung gebildet und daher zickzackförmig. Bei den in den Figuren gezeigten Ausführungsbeispielen von Flächengebilden 3 bestehen diese aus genau einem elektrischen Leiter 5, der zur Abgabe von Kardioversionsimpulsen eingerichtet ist.

Der Leiter 5 weist einen elektrischen Widerstand auf, der kleiner als 150 Ohm, vorzugsweise kleiner als 70 Ohm ist. Auf diese Weise kann mit dem Leiter 5 ein Flächengebilde 3 bereitgestellt werden, das einen für die Kardioversion günstigen elektrischen Widerstand von weniger als 150 Ohm, vorzugsweise von weniger als 70 Ohm aufweist.

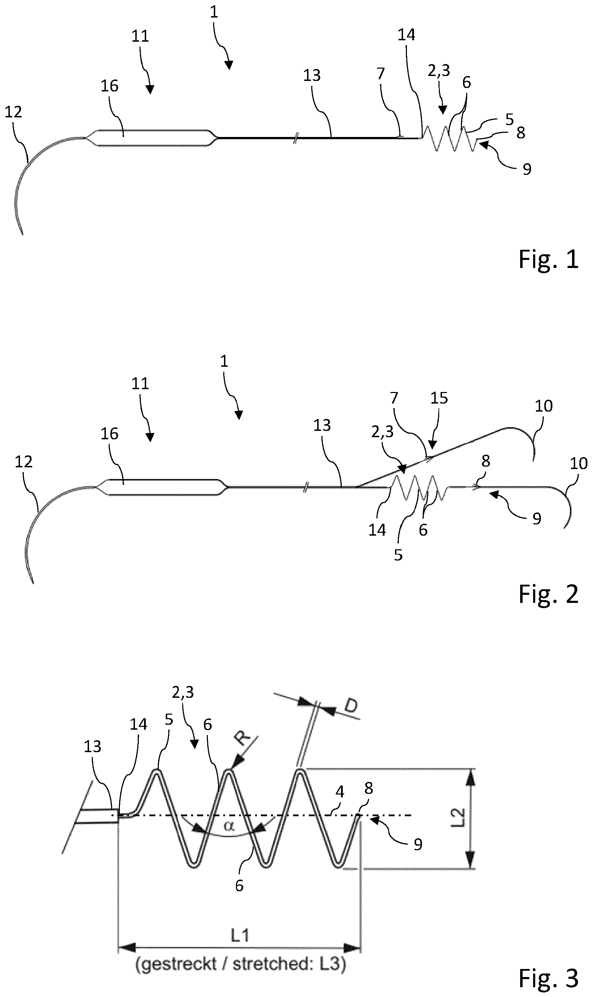

Eine Längsachse des Leiters 5 ist bei unverformtem Flächengebilde 3 in einer Ebene, insbesondere in einer Mittelebene, des Flächengebildes 3 angeordnet und verläuft durch diese Mittelebene mit wechselnder Biegerichtung. Insbesondere die Darstellung aus Figur 3 verdeutlicht, dass das Flächengebilde 3 aus dem mit wechselnder Biegerichtung gebogenen Leiter 5 gebildet ist. Der Leiter 5 weist einen Durchmesser D von 0,1-0,5 mm auf. Der Durchmesser des Leiters 5 kann beispielsweise 0,1-0,3 mm oder auch 2 mm betragen. Der Leiter 5 weist aneinander angrenzende Leiterabschnitte 6 auf, die miteinander verbunden sind und gemeinsam einen Winkel α zwischen 10° und 170° aufspannen.

Bei dem in Figur 3 gezeigten Ausführungsbeispiel beträgt der Winkel α ca. 35°. Die aneinander angrenzenden Leiterabschnitte 6 sind über einen Biegeradius R miteinander verbunden. Der Biegeradius R kann beispielsweise zwischen 0,3 mm bis 2 mm betragen.

Das Flächengebilde 3 kann in nicht aufgespanntem Zustand beispielsweise eine in Richtung seiner Längsachse 4 messbare Längenausdehnung L1 zwischen jeweils einschließlich 10-50 mm aufweisen. In nicht aufgespanntem Zustand weist das Flächengebilde eine quer zu seiner Längsachse 4 messbare Ausdehnung L2 von beispielsweise jeweils einschließlich 5-30 mm auf.

Der elektrische Leiter 5, aus dem das Flächengebilde 3 hergestellt ist, kann eine Länge von beispielsweise 40-140 mm aufweisen. Figur 3 deutet auch eine Längenausdehnung L3 an, die das Flächengebilde 3 in aufgespanntem, gestrecktem Zustand aufweisen kann. Die Längenausdehnung L3 ist dabei größer als die Längenausdehnung L1 und kann ein Mehrfaches der Längenausdehnung L1 des Flächengebildes 3 in nicht aufgespanntem Zustand betragen, beispielsweise das Eineinhalbfache, das Zweifache, das Zweieinhalbfache oder auch das dreifache.

Das Flächengebilde 3 weist als elektrischen Leiter 5 zur Abgabe von Kardioversionsimpulsen eine abisolierte Litze auf. Das Flächengebilde 3, nämlich sein elektrischer Leiter 5 besteht aus Edelstahl.

Bei der in Figur 2 gezeigten, zweiten gezeigten Ausführungsform der Elektrodenanordnung 1 ist erkennbar, dass die Elektrodenanordnung 1 zwei Fixiermittel 7,8 aufweist, zwischen denen das Flächengebilde 3 angeordnet ist. Die Fixiermittel 7,8 dienen dazu, das Flächengebilde 3 an einer Herzaußenseite aufzuspannen und aufgespannt zu befestigen. Das Flächengebilde 3 hat zu zumindest einem der beiden Fixiermittel 7,8 einen Abstand, der zwischen 10 und 70 mm beträgt.

Auch bei der in Figur 1 gezeigten Elektrodenanordnung 1 ist das Flächengebilde 3 zwischen zwei hinsichtlich ihrer Funktion vergleichbaren Fixiermitteln 7 und 8 der Elektrodenanordnung 1 angeordnet. Auch diese Fixiermittel 7 und 8 dienen zur Befestigung und/oder Aufspannung des Flächengebildes 3 an einem Zielgewebe, beispielsweise an einer Herzaußenseite.

Das Flächengebilde 3 ist durch die beiden Fixiermittel 7 und 8 aufspannbar und kann durch das Aufspannen und Strecken des Flächengebildes 3 an eine Vorhofgröße eines zu behandelnden Herzens angepasst werden. Das Flächengebilde 3 kann aufgespannt eine Fläche zwischen 30 mm² und 1600 mm² abdecken. Diese Fläche kann dem Produkt der zuvor erwähnten Ausdehnungen L2 und L3 des aufgespannten und gestreckten Flächengebildes 3 entsprechen.

Das distale Fixiermittel 8 der beiden Fixiermittel 7 und 8 der Elektrodenanordnung 1 ist hierbei distal des Flächengebildes 3, also beispielsweise zwischen einem distalen Ende 9 der Elektrodenanordnung 1 und dem Flächengebilde 3 angeordnet. Die Elektrodenanordnung 1 weist an ihrem distalen Ende 9 ein Verankerungsmittel 10 in Form einer Herznadel auf. Mithilfe des Verankerungsmittels 10 kann die Elektrodenanordnung 1 im Zielgewebe verankert werden. Das Positioniermittel 10 dient dabei dazu, das distale Fixiermittel 8 in seine Gebrauchsstellung im Zielgewebe zu bringen. Danach kann das Positioniermittel 10 abgetrennt und entfernt werden.

An ihrem proximalen Ende 11 weist die Elektrodenanordnung 1 außerdem eine weitere Nadel 12 auf, mit der die Elektrodenanordnung 1, nämlich ihr Anschlussleitung 13, durch die Körperwand eines Patienten gestochen und nach außen geführt werden kann.

Das Flächengebilde 3 ist über die Anschlussleitung 13 mit einem Stimulationsgeber verbindbar. Die Anschlussleitung 13 ist hierbei an einem Punkt 14 mit dem Flächengebilde 3 verbunden, der auf der Längsmittelachse 4 des Flächengebildes 3 liegt. Der Punkt 14 kann auch als Anschlusspunkt bezeichnet werden.

Der Leiter 5 kann ein elektrischer Leiter, insbesondere eine Litze, der Anschlussleitung 13 sein. Das Flächengebilde 3 kann somit aus einem elektrischen Leiter, insbesondere aus einer Litze, der Anschlussleitung 13 bestehen. Der zuvor erwähnte Punkt 14 definiert dann den Übergang zwischen der Anschlussleitung 13 und dem Flächengebilde 3.

An ihrem proximalen Ende 11 weist jede der gezeigten Elektrodenanordnungen 1 ein Anschlussmittel 16 auf, mit dem die jeweilige Anschlussleitung 13 an einen Stimulationsgeber angeschlossen werden kann. Das Anschlussmittel 16 kann beispielsweise ein Anschlussstecker sein.

Die in Figur 2 gezeigte Elektrodenanordnung 1 weist zusätzlich zu der Kardioversionselektrode 2 eine implantierbare Lateral-Elektrode 15 auf. Die Lateral-Elektrode 15 dient der Abgabe von Pacing-Impulsen auf das Zielgewebe. Die Abgabe von Pacing-Impulsen kann hierbei im Zusammenspiel mit der Kardioversionselektrode 2 erfolgen.

Der Lateral-Elektrode 15 der in Figur 2 gezeigten Elektrodenanordnung 1 ist das Fixiermittel 7 der beiden Fixiermittel 7 und 8 zugeordnet. Das Fixiermittel 7 wird somit auch zur Befestigung der Lateral-Elektrode 15 in einem Zielgewebe verwendet. Das der Lateral-Elektrode 15 zugeordnete Fixiermittel 7 ist somit eines der beiden Fixiermittel 7 und 8, das der Befestigung und/oder Aufspannung des Flächengebildes 3 dient. Dieses Fixiermittel 7 kann mithilfe eines Verankerungsmittels 10, das distal der Lateral-Elektrode 15 angeordnet ist, in das Zielgewebe eingebracht werden. Auch dieses Positioniermittel 10 ist als Herznadel ausgebildet.

Figur 2 deutet an, dass die Lateral-Elektrode 15 und die zumindest eine Kardioversionselektrode 2, nämlich das Flächengebilde 3, in einem frei wählbaren Winkel relativ zueinander angeordnet werden können, beispielsweise in einem Winkel zwischen 1° und 170°. Die Anordnung der Lateral-Elektrode 15 und der Kardioversionselektrode 2 in einem Winkelbereich von 1° bis 170° und vorzugsweise in einem Winkel zueinander, der kleiner als 90° ist, kann die schonende und vorzugsweise atraumatische Explanation der Elektrodenanordnung begünstigen. Die Lateral-Elektrode 15, die Kardioversionselektrode 2 und die Anschlussleitung 13 bilden dann eine Y-Form.

Die Elektrodenanordnung 1, nämlich die Kardioversionselektrode 2 und die Lateral-Elektrode 15 können durch Zug an dem proximalen Ende 11 der Elektrodenanordnung 1, nämlich durch Zug am proximalen Ende 11 der Anschlussleitung 13 der Elektrodenanordnung 1 explantiert werden. Auch die in Figur 1 gezeigte Elektrodenanordnung 1 kann auf dieselbe Art und Weise durch Zug an der Anschlussleitung 13 explantiert werden.

Die Erfindung befasst sich mit Verbesserungen auf dem technischen Gebiet der elektromedizinischen Elektrodenanordnungen. Als Verbesserung wird eine elektromedizinische Elektrodenanordnung 1 vorgeschlagen, die zumindest eine implantierbare Kardioversionselektrode 2 aufweist, die aus einem flexiblen, in unverformter Gebrauchsstellung ebenen, elektrisch leitenden Flächengebilde 3 besteht, das zur flächigen Anlage an ein Zielgewebe eingerichtet ist.

### Bezugszeichenliste

- 1: elektromedizinische Elektrodenanordnung
- 2: Kardioversionselektrode
- 3: Flächengebilde
- 4: Längsachse von 3
- 5: longitudinale Struktur, elektrischer Leiter
- 6: angrenzende Leiterabschnitte
- 7: Fixiermittel
- 8: distales Fixiermittel
- 9: distales Ende von 1
- 10: Positioniermittel, Herznadel
- 11: proximales Ende
- 12: Nadel an 11
- 13: Anschlussleitung
- 14: Punkt
- 15: Lateral-Elektrode
- 16: Anschlussmittel

## Patentansprüche

1. Elektromedizinische Elektrodenanordnung (1) mit zumindest einer implantierbaren Kardioversionselektrode (2) zur Abgabe von Kardioversionsimpulsen auf ein Zielgewebe, insbesondere auf Herzmuskelgewebe, **dadurch gekennzeichnet, dass** die Kardioversionselektrode (2) aus einem flexiblen, in unverformter Ausgangsstellung ebenen, elektrisch leitenden Flächengebilde (3) besteht, das zur flächigen Anlage an ein Zielgewebe eingerichtet und bestimmt ist, wobei die Elektrodenanordnung (1) zwei Fixiermittel (7,8) aufweist, zwischen denen das Flächengebilde (3) angeordnet ist und die zur Befestigung und/oder Aufspannung des Flächengebildes (3) an einem Zielgewebe, beispielsweise an einer Herzaußenseite, eingerichtet sind.

2. Elektrodenanordnung (1) nach dem vorherigen Anspruch, wobei das Flächengebilde (3) elastisch ist.

3. Elektrodenanordnung (1) nach einem der vorherigen Ansprüche, wobei das Flächengebilde (3) eine Längsachse (4), insbesondere eine Längsmittelachse, aufweist, die bei unverformtem Flächengebilde (3) in einer Ebene verläuft.

4. Elektrodenanordnung (1) nach einem der vorherigen Ansprüche, wobei das Flächengebilde (3) aus einer longitudinalen Struktur (5), insbesondere aus einem elektrischen Leiter, mit wechselnder Biegerichtung besteht und/oder sinusförmig ist.

5. Elektrodenanordnung (1) nach einem der vorherigen Ansprüche, wobei das Flächengebilde (3) aus zumindest einem, insbesondere aus genau einem, elektrischen Leiter (5) zur Abgabe von Kardioversionsimpulsen gebildet ist.

6. Elektrodenanordnung (1) nach dem vorherigen Anspruch, wobei der zumindest eine elektrische Leiter (5) einen elektrischen Widerstand aufweist, der kleiner als 150 Ohm, vorzugsweise kleiner als 70 Ohm ist.

7. Elektrodenanordnung (1) nach einem der beiden vorherigen Ansprüche, wobei eine Längsachse des zumindest einen Leiters (5) in einer Ebene des unverformten Flächengebildes (3) und/oder mit wechselnder Biegerichtung verläuft.

8. Elektrodenanordnung (1) nach einem der Ansprüche 5 bis 7, wobei der zumindest eine Leiter (5) einen Durchmesser D von 0,1 bis 0,5mm, insbesondere 0,1 bis 0,3 mm, insbesondere von 0,2 mm aufweist.

9. Elektrodenanordnung (1) nach einem der Ansprüche 5 bis 8, wobei der Leiter (5) zumindest zwei aneinander angrenzende und/oder miteinander verbundene Leiterabschnitte (6) aufweist, die gemeinsam einen Winkel α zwischen 10° und 170° aufspannen und/oder über einen Biegeradius R miteinander verbunden sind, insbesondere durch einen Biegeradius von 0,3 bis 2mm.

10. Elektrodenanordnung (1) nach einem der vorherigen Ansprüche, wobei das Flächengebilde (3) in nicht aufgespanntem Zustand eine in Richtung seiner Längsachse (4) messbare Längenausdehnung L1 zwischen jeweils einschließlich 10 bis 50 mm, insbesondere zwischen jeweils einschließlich 25 bis 40 mm, hat.

11. Elektrodenanordnung (1) nach einem der vorherigen Ansprüche, wobei das Flächengebilde (3) in nicht aufgespanntem Zustand eine quer zu seiner Längsachse (4) messbare Ausdehnung L2 von jeweils einschließlich 5 bis 30 mm hat, und/oder aus zumindest einem elektrischen Leiter (5) gebildet ist, dessen Länge 40 bis 140 mm beträgt.

12. Elektrodenanordnung (1) nach einem der Ansprüche 5-11, wobei der zumindest eine elektrischen Leiter (5) eine abisolierte Litze zur Abgabe von Kardioversionsimpulsen ist.

13. Elektrodenanordnung (1) nach einem der Ansprüche 5-12, wobei das Flächengebilde (3), insbesondere sein elektrischer Leiter (5), aus Edelstahl, aus Platin, aus einer Platinlegierung, aus Gold, aus einer Goldlegierung, aus Magnesium, aus einer Magnesiumlegierung, aus Molybdän oder aus einer Molybdänlegierung besteht.

14. Elektrodenanordnung (1) nach dem einem der vorherigen Ansprüche, wobei das Flächengebilde (3) zu zumindest einem den beiden Fixiermittel (7,8) einen Abstand zwischen einschließlich 10 und 70 mm aufweist.

15. Elektrodenanordnung (1) nach einem der vorherigen Ansprüche, wobei das Flächengebilde (3), insbesondere durch die Fixiermittel (7,8), aufspannbar und/oder an eine Vorhofgröße eines zu behandelnden Herzens anpassbar ist.

16. Elektrodenanordnung (1) nach einem der vorherigen Ansprüche, wobei ein distales Fixiermittel (8) der Elektrodenanordnung (1) zur Befestigung und/oder Aufspannung des Flächengebildes (3) distal des Flächengebildes (3), insbesondere an einem distalen Ende (9) der Elektrodenanordnung (1) oder zwischen einem distalen Ende (9) der Elektrodenanordnung (1) und dem Flächengebilde (3), angeordnet ist.

17. Elektrodenanordnung (1) nach einem der vorherigen Ansprüche, wobei die Elektrodenanordnung (1) an ihrem distalen Ende (9) zumindest ein Positioniermittel (10), insbesondere eine Herznadel, zur Positionierung eines Fixiermittels (8) der Elektrodenanordnung (1) im Zielgewebe aufweist.

18. Elektrodenanordnung (1) nach einem der vorherigen Ansprüche, wobei die Elektrodenanordnung (1) eine Anschlussleitung (13) aufweist, über den das Flächengebilde (3) mit einem Stimulationsgeber verbindbar ist, insbesondere wobei die Anschlussleitung (13) an einem Punkt (14) mit dem Flächengebilde (3) verbunden ist, der auf einer Längsmittelachse (4) des Flächengebildes (3) liegt.

19. Elektrodenanordnung (1) nach einem der vorherigen Ansprüche, wobei die Elektrodenanordnung (1) zumindest eine implantierbare Lateral-Elektrode (15) zur Abgabe von Pacing-Impulsen aufweist.

20. Elektrodenanordnung (1) nach dem vorherigen Anspruch, wobei der zumindest einen Lateral-Elektrode (15) zumindest ein Fixiermittel (7,8) zur Befestigung der Lateral-Elektrode (15) in einem Zielgewebe zugeordnet ist.

21. Elektrodenanordnung (1) nach dem vorherigen Anspruch, wobei das der Lateral-Elektrode (15) zugeordnete Fixiermittel (7,8) ein Fixiermittel von zumindest zwei Fixiermitteln (7,8) zur Befestigung und/oder zur Aufspannung des Flächengebildes (2) ist.

22. Elektrodenanordnung (1) nach einem der vorherigen Ansprüche, wobei die zumindest eine Lateral-Elektrode (15) und die zumindest eine Kardioversionselektrode (2) in einem freiwählbaren Winkel relativ zueinander anordenbar sind, insbesondere in einem Winkel zwischen 1° und 170°.

23. Elektrodenanordnung (1) nach dem Anspruch 19, wobei die Elektrodenanordnung (1), insbesondere die zumindest eine Kardioversionselektrode (2) und/oder die zumindest eine Lateral-Elektrode (15), durch Zug an einem proximalen Ende (11) der Elektrodenanordnung (1) explantierbar ist.

## Claims

1. Electromedical electrode assembly (1) having at least one implantable cardioversion electrode (2) for delivering cardioversion pulses to a target tissue, in particular to heart muscle tissue, **characterized in that** the cardioversion electrode (2) consists of a flexible, electrically conductive sheet-like structure (3) which is planar in the undeformed initial position and which is designed and intended for flat application to a target tissue, wherein the electrode assembly (1) has two fixing means (7, 8), between which the sheet-like structure (3) is arranged and which are designed for fastening and/or clamping the sheet-like structure (3) to a target tissue, for example to an outside of the heart.

2. Electrode assembly (1) according to the preceding claim, wherein the sheet-like structure (3) is elastic.

3. Electrode assembly (1) according to one of the preceding claims, wherein the sheet-like structure (3) has a longitudinal axis (4), in particular a longitudinal center axis, which extends in a plane when the sheet-like structure (3) is not deformed.

4. Electrode assembly (1) according to one of the preceding claims, wherein the sheet-like structure (3) consists of a longitudinal structure (5), in particular of an electrical conductor, with an alternating bending direction and/or is sinusoidal.

5. Electrode assembly (1) according to one of the preceding claims, wherein the sheet-like structure (3) is formed from at least one, in particular exactly one, electrical conductor (5) for the delivery of cardioversion pulses.

6. Electrode assembly (1) according to the preceding claim, wherein the at least one electrical conductor (5) has an electrical resistance which is less than 150 ohms, preferably less than 70 ohms.

7. Electrode assembly (1) according to one of the two preceding claims, wherein a longitudinal axis of the at least one conductor (5) extends in a plane of the non-deformed sheet-like structure (3) and/or with an alternating bending direction.

8. Electrode assembly (1) according to one of claims 5 to 7, wherein the at least one conductor (5) has a diameter D of 0.1 to 0.5 mm, in particular 0.1 to 0.3 mm, in particular 0.2 mm.

9. Electrode assembly (1) according to one of claims 5 to 8, wherein the conductor (5) has at least two mutually adjoining and/or interconnected conductor sections (6) which together span an angle α between 10° and 170° and/or are interconnected via a bending radius R, in particular via a bending radius of 0.3 to 2 mm.

10. Electrode assembly (1) according to one of the preceding claims, wherein the sheet-like structure (3), in the non-clamped state, has a linear extension L1, measurable in the direction of its longitudinal axis (4), of between 10 to 50 mm, in particular between 25 to 40 mm, in each case inclusive.

11. Electrode assembly (1) according to one of the preceding claims, wherein the sheet-like structure (3), in the non-clamped state, has an extension L2, measurable transversely to its longitudinal axis (4), of between 5 and 30 mm, inclusive, and/or is formed from at least one electrical conductor (5), the length of which is 40 to 140 mm.

12. Electrode assembly (1) according to one of the preceding claims 5 to 11, wherein the at least one electrical conductor (5) is a bared strand for delivering cardioversion pulses.

13. Electrode assembly (1) according to one of the claims 5 to 12, wherein the sheet-like structure (3), in particular its electrical conductor (5), consists of stainless steel, of platinum, of a platinum alloy, of gold, of a gold alloy, of magnesium, of a magnesium alloy, of molybdenum or of a molybdenum alloy.

14. Electrode assembly (1) according to one of the preceding claims, wherein the sheet-like structure (3) is at a distance of between 10 and 70 mm, inclusive, from at least one of the two fixing means (7, 8).

15. Electrode assembly (1) according to one of the preceding claims, wherein the sheet-like structure (3) can be clamped, in particular by the fixing means (7, 8), and/or adapted to the atrial size of a heart to be treated.

16. Electrode assembly (1) according to one of the preceding claims, wherein a distal fixing means (8) of the electrode assembly (1) for fastening and/or clamping the sheet-like structure (3) is distal of the sheet-like structure (3), in particular at a distal end (9) of the electrode assembly (1) or between a distal end (9) of the electrode assembly (1) and the sheet-like structure (3).

17. Electrode assembly (1) according to one of the preceding claims, wherein the electrode assembly (1) has at its distal end (9) at least one positioning means (10), in particular a heart needle, for positioning a fixing means (8) of the electrode assembly (1) in the target tissue.

18. Electrode assembly (1) according to one of the preceding claims, wherein the electrode assembly (1) has a connecting lead (13) by means of which the sheet-like structure (3) can be connected to a stimulation generator , in particular wherein the connecting lead (13) is connected to the sheet-like structure (3) at a point (14) which lies on a longitudinal center axis (4) of the sheet-like structure (3).

19. Electrode assembly (1) according to one of the preceding claims, wherein the electrode assembly (1) comprises at least one implantable lateral electrode (15) for delivering pacing pulses.

20. Electrode assembly (1) according to the preceding claim, wherein at least one lateral electrode (15) is associated with at least one fixing means (7, 8) for securing the lateral electrode (15) in a target tissue.

21. Electrode assembly (1) according to the preceding claim, wherein the fixing means (7, 8) associated with the lateral electrode (15) is one of at least two fixing means (7, 8) for fastening and/or clamping the sheet-like structure (2).

22. Electrode assembly (1) according to one of the preceding claims, wherein the at least one lateral electrode (15) and the at least one cardioversion electrode (2) can be arranged at a freely selectable angle relative to one another, in particular at an angle between 1° and 170°.

23. Electrode assembly (1) according to claim 19, wherein the electrode assembly (1), in particular the at least one cardioversion electrode (2) and/or the at least one lateral electrode (15), can be explanted by pulling on a proximal end (11) of the electrode assembly (1).

## Revendications

1. Ensemble d'électrodes électromédicales (1) avec au moins une électrode de cardioversion implantable (2) pour délivrer des impulsions de cardioversion sur un tissu cible, notamment sur un tissu de muscle cardiaque, **caractérisé en ce que** l'électrode de cardioversion (2) est constituée d'un matériau en feuille textile (3) électriquement conducteur flexible qui est plat dans une position initiale non déformée et qui est conçu et destiné à être appliqué à plat sur un tissu cible, l'ensemble d'électrodes (1) présentant deux moyens de fixation (7, 8) entre lesquels est disposé le matériau en feuille textile (3) et qui sont conçus pour fixer et/ou serrer le matériau en feuille textile (3) sur un tissu cible, par exemple sur un côté extérieur du coeur.

2. Ensemble d'électrodes (1) selon la revendication précédente, le matériau en feuille textile (3) étant élastique.

3. Ensemble d'électrodes (1) selon l'une des revendications précédentes, le matériau en feuille textile (3) présentant un axe longitudinal (4), notamment un axe médian longitudinal, qui s'étend dans un plan lorsque le matériau en feuille textile (3) n'est pas déformé.

4. Ensemble d'électrodes (1) selon l'une des revendications précédentes, le matériau en feuille textile (3) étant constitué d'une structure longitudinale (5), notamment d'un conducteur électrique, à sens de courbure alterné et/ou étant sinusoïdale.

5. Ensemble d'électrodes (1) selon l'une des revendications précédentes, le matériau en feuille textile (3) étant formé d'au moins un, notamment d'exactement un, conducteur électrique (5) pour délivrer des impulsions de cardioversion.

6. Ensemble d'électrodes (1) selon la revendication précédente, l'au moins un conducteur électrique (5) présentant une résistance électrique qui est inférieure à 150 ohms, de préférence inférieure à 70 ohms.

7. Ensemble d'électrodes (1) selon l'une des deux revendications précédentes, un axe longitudinal de l'au moins un conducteur (5) s'étendant dans un plan du matériau en feuille textile (3) non déformé et/ou à sens de courbure alterné.

8. Ensemble d'électrodes (1) selon l'une des revendications 5 à 7, l'au moins un conducteur (5) présentant un diamètre D compris entre 0,1 et 0,5 mm, notamment entre 0,1 et 0,3 mm, notamment de 0,2 mm.

9. Ensemble d'électrodes (1) selon l'une des revendications 5 à 8, le conducteur (5) présentant au moins deux sections de conducteur (6) adjacentes et/ou reliées entre elles, qui forment ensemble un angle α compris entre 10° et 170° et/ou qui sont reliées entre elles par un rayon de courbure R, notamment par un rayon de courbure compris entre 0,3 et 2 mm.

10. Ensemble d'électrodes (1) selon l'une des précédentes revendications, le matériau en feuille textile (3) ayant, à l'état non serré, une extension longitudinale L1 mesurable dans la direction de son axe longitudinal (4), comprise entre respectivement 10 et 50 mm inclus, notamment entre respectivement 25 et 40 mm inclus.

11. Ensemble d'électrodes (1) selon l'une des revendications précédentes, le matériau en feuille textile (3) ayant, à l'état non serré, une extension L2 mesurable transversalement à son axe longitudinal (4) de respectivement 5 à 30 mm inclus, et/ou étant formée d'au moins un conducteur électrique (5) dont la longueur est de 40 à 140 mm.

12. Ensemble d'électrodes (1) selon l'une des revendications 5-11, l'au moins un conducteur électrique (5) étant un toron dénudé pour délivrer des impulsions de cardioversion.

13. Ensemble d'électrodes (1) selon l'une des revendications 5-12, le matériau en feuille textile (3), notamment son conducteur électrique (5), étant en acier inoxydable, en platine, en alliage de platine, en or, en alliage d'or, en magnésium, en alliage de magnésium, en molybdène ou en alliage de molybdène.

14. Ensemble d'électrodes (1) selon l'une des revendications précédentes, le matériau en feuille textile (3) étant espacé d'au moins un des deux moyens de fixation (7, 8) d'une distance comprise entre 10 et 70 mm.

15. Ensemble d'électrodes (1) selon l'une des revendications précédentes, le matériau en feuille textile (3) étant serrable, notamment par les moyens de fixation (7, 8), et/ou adaptable à une taille d'oreillette d'un coeur à traiter.

16. Ensemble d'électrodes (1) selon l'une des revendications précédentes, un moyen de fixation distal (8) de l'ensemble d'électrodes (1) pour fixer et/ou serrer le matériau en feuille textile (3) étant disposé distalement au matériau en feuille textile (3), notamment à une extrémité distale (9) de l'ensemble d'électrodes (1) ou entre une extrémité distale (9) de l'ensemble d'électrodes (1) et le matériau en feuille textile (3).

17. Ensemble d'électrodes (1) selon l'une des revendications précédentes, l'ensemble d'électrodes (1) présentant à son extrémité distale (9) au moins un moyen de positionnement (10), notamment une aiguille cardiaque, pour positionner un moyen de fixation (8) de l'ensemble d'électrodes (1) dans le tissu cible.

18. Ensemble d'électrodes (1) selon l'une des revendications précédentes, l'ensemble d'électrodes (1) présentant une ligne de raccordement (13) par laquelle le matériau en feuille textile (3) est reliable à un générateur de stimulation, notamment la ligne de raccordement (13) étant reliée au matériau en feuille textile (3) en un point (14) qui se trouve sur un axe médian longitudinal (4) du matériau en feuille textile (3).

19. Ensemble d'électrodes (1) selon l'une des revendications précédentes, l'ensemble d'électrodes (1) présentant au moins une électrode latérale implantable (15) pour délivrer des impulsions de stimulation.

20. Ensemble d'électrodes (1) selon la revendication précédente, au moins un moyen de fixation (7, 8) étant associé à l'au moins une électrode latérale (15) pour fixer l'électrode latérale (15) dans un tissu cible.

21. Ensemble d'électrodes (1) selon la revendication précédente, le moyen de fixation (7, 8) associé à l'électrode latérale (15) étant un moyen de fixation d'au moins deux moyens de fixation (7, 8) pour fixer et/ou serrer le matériau en feuille textile (2).

22. Ensemble d'électrodes (1) selon l'une des revendications précédentes, l'au moins une électrode latérale (15) et l'au moins une électrode de cardioversion (2) sont disposables l'une par rapport à l'autre selon un angle pouvant être librement choisi, notamment selon un angle compris entre 1° et 170°.

23. Ensemble d'électrodes (1) selon la revendication 19, l'ensemble d'électrodes (1), notamment l'au moins une électrode de cardioversion (2) et/ou l'au moins une électrode latérale (15), étant explantable par traction sur une extrémité proximale (11) de l'ensemble d'électrodes (1).
